(19) Europäisches Patentamt
European Patent Office
Office européen des brevets

(11) Publication number: **0 125 004**
**B1**

(12) **EUROPEAN PATENT SPECIFICATION**

(45) Date of publication of patent specification: **08.04.87**

(51) Int. Cl.[4]: **A 61 K 31/71,** A 61 K 31/63 // (A61K31/71, 31:63, 31:35)

(21) Application number: **84302242.7**

(22) Date of filing: **02.04.84**

(54) **Synergistic antiparasitic compositions.**

(30) Priority: **07.04.83 US 483045**

(43) Date of publication of application:
**14.11.84 Bulletin 84/46**

(45) Publication of the grant of the patent:
**08.04.87 Bulletin 87/15**

(84) Designated Contracting States:
**CH DE FR GB IT LI NL**

(56) References cited:
**US-A-4 064 239**
**US-A-4 310 519**

(73) Proprietor: **MERCK & CO. INC.**
**126, East Lincoln Avenue P.O. Box 2000**
**Rahway New Jersey 07065 (US)**

(72) Inventor: **Campbell, William C.**
**49 Club Drive**
**Summit New Jersey 07901 (US)**
Inventor: **Fisher, Michael H.**
**R.D. 1, Box 302 Old York Road**
**Ringoes New Jersey 08551 (US)**

(74) Representative: **Crampton, Keith John Allen et al**
**D YOUNG & CO 10 Staple Inn**
**London WC1V 7RD (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 125 004 B1

## Description

Avermectin compounds are a series of natural products isolated from the fermentation broth of a strain of *Streptomyces avermitilis.* The series consists of eight compounds, four major and four minor. The compounds are disclosed in US Patent Specification No US—A—4,310,519. Certain derivatives of such compounds are also disclosed, such as the 22,23-dihydro derivatives described in US Patent Specification No US—A—4,199,569. The 13-deoxy derivatives of avermectin compounds are disclosed in US Patent Specifications Nos US—A—4,171,314 and US—A—4,173,571. In addition, the 4″-phosphate derivatives of the avermectin compounds with a 13-O-disaccharide group are disclosed in copending US Patent Application Serial No 461,843.

When used as antiparasitic agents the avermectin compounds are administered at dosage rates of from 0.001 to 10 mg of the active compound per kg of weight of the host animal.

Clorsulon is disclosed in US Patent Specification No US—A—4,064,239. When used as fasciolicidal antiparasitic agents the clorsulon compound is administered at dosage rates of from 1 to 25 mg of the active compound per kg of weight of the host animal.

The present invention provides a mixture of an avermectin compound having the formula set forth below and clorsulon, which has a synergistic effect when administered to animals for the treatment of parasitic diseases. The avermectin compounds used in the compositions of this invention having the following formula:

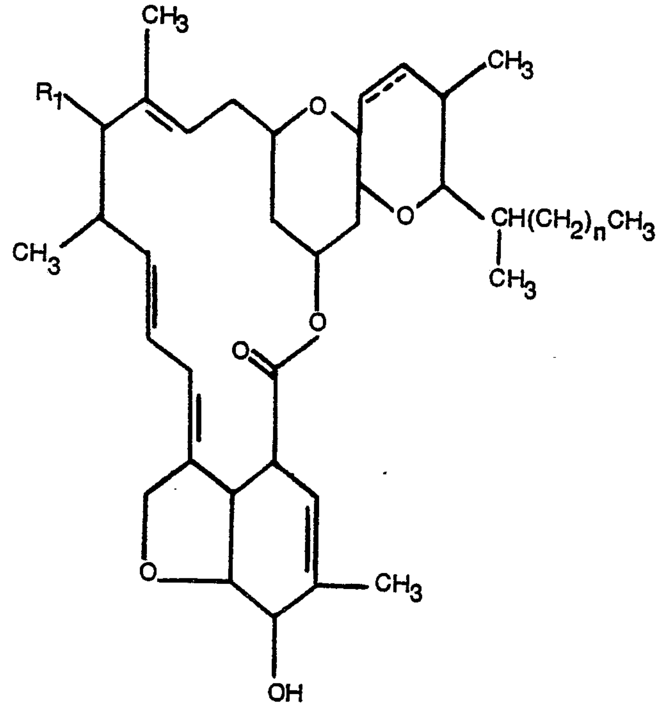

where *n* is 0 or 1;

$R_1$ is hydrogen or α-*L*-oleandrosyl-α-L-oleandrosyloxy or its 4″-phosphate derivative; and the broken line indicates a single or a double bond.

The clorsulon compound used in the compositions of the present invention has the formula:

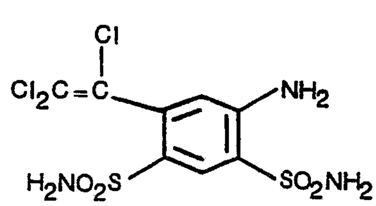

The synergistic effect of the mixture of avermectin compound with clorsulon is observed in that a reduced dosage of one or both of the components as required, compared with that stated above. Thus, a lessened quantity of the antiparasitic compound is administered than normally would be required, which results in a lessening of possible side effects and a lessening in the development of resistance. In addition, there is a synergistic expansion of the spectrum of parasitic infections that may be successfully combated, compared with consideration of the spectra of activity of the individual components. Thus, the possibility of

eliminating parasitic infections against which the individual components are ineffective or only partially effective is realized in the composition of the present invention.

The parasitic infections against which compositions of the present invention have been found particularly effective are flatworms of the *Fasciola* species, as well as other gastrointestinal worms found in dogs, cats, sheep, cattle, horses, pigs and other animals.

In the compositions of the present invention, the proportions of the individual components may be 1 part by weight of the avermectin compound to from 1 to 1000 parts by weight of clorsulon.

The synergistic combination may be administered orally in unit dosage form such as a capsule, bolus or tablet, or as a liquid drench when used as an antiparasitic in mammals. The drench is normally a solution, suspension or dispersion of the active ingredients, usually in water, together with a suspending agent such as bentonite and a wetting agent or like excipient. Generally, the drenches also contain an antifoaming agent. Drench formulations generally contain from 0.001 to 0.5%, preferably 0.01 to 1%, by weight of the active compounds. The capsules and boluses comprise the active ingredients admixed with a carrier vehicle such as starch, talc, magnesium stearate, or dicalcium phosphate.

Where it is desired to administer the composition in a dry, solid unit-dosage form, capsules, boluses or tablets containing the desired amount of active compounds are usual. These dosage forms are prepared by intimately and uniformly mixing the active ingredients with suitable finely divided diluents, fillers, disintegrating agents and/or binders such as starch, lactose, talc, magnesium stearate and vegetable gums. Such unit disage formulations may vary widely in total weight and content of the antiparasitic agent, depending upon factors such as the type of holst animal to be treated, the severity and type of infection, and the weight of the host.

When the composition of the present invention is to be administered in an animal feedstuff, it is intimately dispersed in the feed or used as a top dressing or in the form of pellets, which may then be added to the finished feed or optionally fed separately. Alternatively, it may be administered to animals parenterally, for example, by intraruminal, intramuscular, intratracheal, or subcutaneous injection, in which case the active ingredient is dissolved or dispersed in a liquid carrier vehicle. For parenteral administration, the active material is suitably admixed with an acceptable vehicle, preferably of the vegetable oil variety such as peanut oil or cotton-seed oil. Other parenteral vehicles such as organic preparations using solketal, glycerol, formal and aqueous parenteral formulations are also used. The active compounds are dissolved or suspended in the parenteral formulation for administrations; such formulations generally contain from 0.005 to 5% by weight of the active compound.

Specific formulations containing avermectin compounds and clorsulon compounds which have synergistic antiparasitic effects are as follows:

A bolus is made, consisting of 2.5 g starch, 2.5 g lactose, 100 mg clorsulon and 10 mg ivermectin. The bolus is administered, by means of a balling gun, to a calf weighing 100 kg body weight and harbouring parasites of the genera *Haemonchus, Trichostrongylus, Ostertagia, Parafilaria, Damalinia, Amblyomma, Linognathus* and *Sarcoptes*. The treatment results in a high degree of efficacy against the said parasite species.

In addition, an oral drench or a feed supplement may be prepared containing the active ingredients in quantities sufficient to deliver ivermectin at 0.2 mg/kg and clorsulon at 1.0 mg/kg.

A solution or suspension or other formulation suitable for parenteral administration may be prepared containing the active ingredients in quantities sufficient to provide ivermectin at a dosage of 0.2 mg/kg and clorsulon at 1.0 mg/kg.

**Claims**

1. A composition comprising 4-amino-6-trichlorovinyl-1,3-benzenedisulfonamide (clorsulon) and an avermectin compound having the formula:

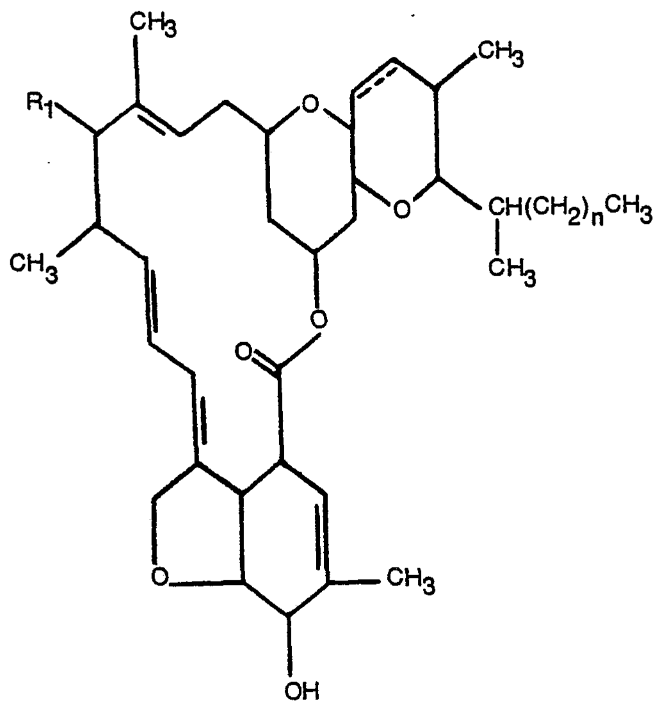

where *n* is 0 or 1;

$R^1$ is hydrogen or α-*L*-oleandrosyl-α-L-oleandrosyloxy or its 4″-phosphate derivative; and the broken line indicates a single or a double bond.

2. A composition as claimed in claim 1 containing 1 part by weight of the avermectin compound to form 1 to 1000 parts by weight of clorsulon.

3. An antiparasitic formulation comprising an inert carrier and from 0.001 to 5% by weight of a composition as claimed in claim 1.

4. A composition as claimed in claim 1 for use in the treatment of parasitic infections in animals.

**Patentansprüche**

1. Zusammensetzung, enthaltend 4-Amino-6-trichlorvinyl-1,3-benzoldisulfonamid (Clorsulon) und eine Avermectin-Verbindung mit der Formel

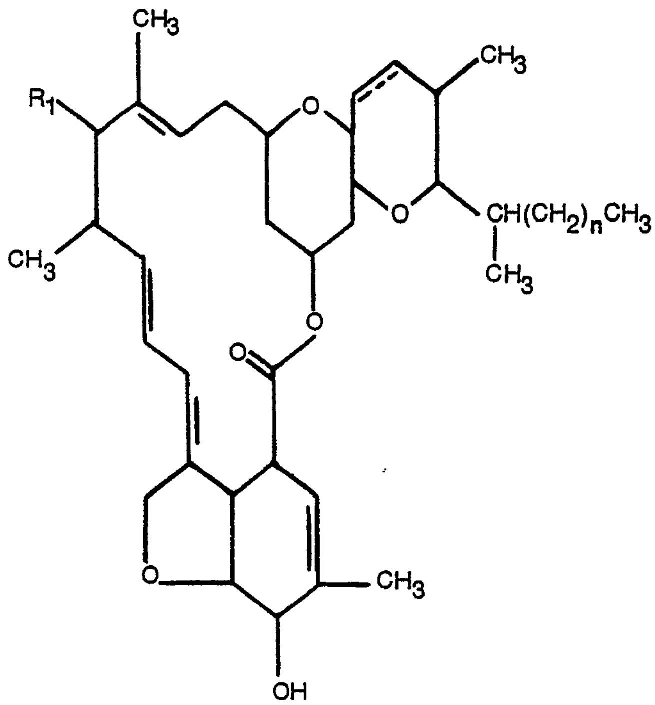

worin *n* 0 oder 1 ist;

$R_1$ Wasserstoff oder α-*L*-Oleandrosyl-α-L-oleandrosyloxy oder sein 4″-Phosphatderivat ist; und die unterbrochene Linie eine Einfachbindung oder eine Droppelbindung anzeigt.

2. Zusammensetzung nach Anspruch 1, enthaltend 1 Gew.-Teil der Avermectin-Verbindung auf 1 bis 1000 Gew.-Teile Clorsulon.

3. Antiparasitäre Formulierung, enthaltend eine inerten Träger und 0,001 bis 5 Gew.-% einer Zusammensetzung nach Anspruch 1.

4. Zusammensetzung nach Anspruch 1 zur Verwendung bei der Behandlung parasitärer Infektionen bei Tieren.

**Revendications**

1. Une composition comprenant du 4-amino-6-trichlorovinyl-1,3-benzènedisulfonamide (clorsulon) et une avermectine de formule:

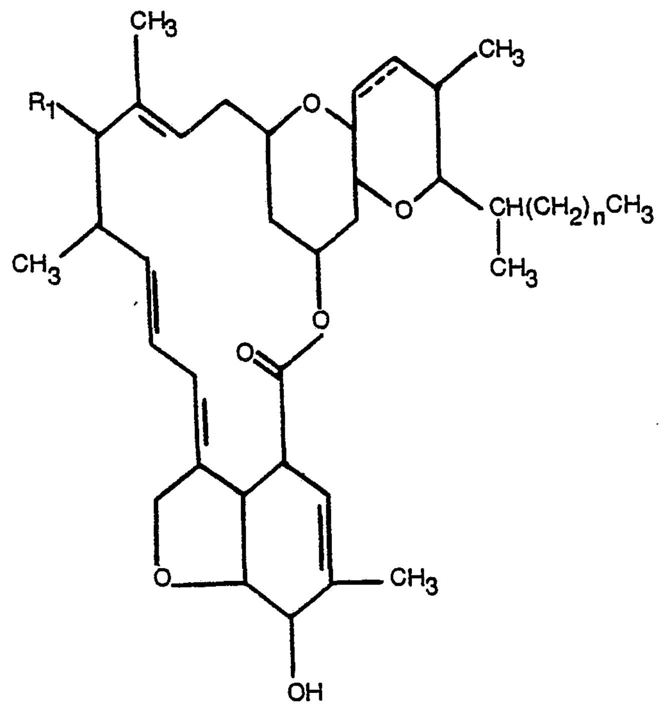

dans laquelle
n est 0 ou 1;
$R_1$ est un hydrogène ou un α-L-oléandrosyl-α-L-oléandrosyl oxy ou son dérivé de type 4″-phosphate; et
le pointillé indique une simple liaison ou une double liaison.

2. Une composition comme revendiquée dans la revendication 1, contenant 1 partie en poids de l'avermectine et de 1 à 1000 parties en poids de clorsulon.

3. Une composition antiparasitaire comprenant un support inerte et de 0,001 à 5 % en poids d'une composition comme revendiquée dans la revendication 1.

4. Une composition comme revendiquée dans la revendication 1, pour l'emploi dans le traitement des infections parasitaires des animaux.